# EUROPEAN PATENT APPLICATION

(11) **EP 4 793 635 A1**
(43) Date of publication of application: **19.08.2026**
(21) Application number: 25157745.8
(22) Date of filing: 13.02.2025
(51) Int. Cl.: G01N 21/95

(54) **IMAGING DEVICE AND METHOD FOR DETECTING A SEAM AREA BETWEEN MEDICATION SACHETS**

(71) Applicant: Evondos Oy, 24240 Salo (FI)
(72) Inventor: JONES, Steven, Royston, SG8 5GA (GB); NEWMAN, Matthew, Cambridgeshire, CB24 3AL (GB)
(74) Representative: Berggren Oy

(57) **Abstract**

The present invention relates to an imaging device (100) that comprises a first polarizer (101) and a second polarizer (102) between which a strip (200) of medication sachets can be arranged, a camera (103) for imaging a first side of the strip (200) of medication sachets through the second polarizer (102), and means (104) for changing the polarization direction of the first polarizer (101) or the second polarizer (102). The present invention also relates to a method for detecting a seam area (203) between medication sachets (201, 202) and to a medication dispenser (300).

## Description

### TECHNICAL FIELD OF THE INVENTION

The present invention relates to an imaging device and to a method for detecting a seam area between medication sachets according to the preambles of the appended independent claims. The invention also relates to a medication dispenser.

### BACKGROUND OF THE INVENTION

Various medication dispensers are known for automated dispensing of medications to a patient. Many of these medication dispensers use strips of prepackaged medication sachets, where each medication sachet contains the medications that the patient should take at a prescribed date and time. The medication dispenser separates the medication sachets from the strip and dispenses them to the patient one medication sachet at a time.

A medication sachet to be dispensed is separated from a strip of medication sachets by using a cutting device. The cutting device cuts the strip in a transverse direction at a location that is typically determined with an imaging device. The strip of medication sachets should be cut along a seam area that is located between the medication sachets.

A problem associated with the known imaging devices is that in many cases the location of a seam area cannot be detected accurately, and as a result the strip of medication sachets is cut at a wrong location. In the worst case, the medication sachet is cut in half whereby the medications may fall out of the medication sachet and drop inside the medication dispenser.

### OBJECTIVES OF THE INVENTION

It is the main objective of the present invention to reduce or even eliminate the prior art problems presented above.

It is an objective of the present invention to provide an imaging device for a medication dispenser. In more detail, it is an objective of the invention to provide an imaging device enabling to accurately detect a seam area between medication sachets.

It is also an objective of the present invention to provide a method enabling to accurately detect a seam area between medication sachets.

It is also an objective of the present invention to provide a medication dispenser for dispensing medications to a patient.

In order to realise the above-mentioned objectives, the imaging device and the method according to the invention are characterised by what is presented in the characterising portions of the appended independent claims. Advantageous embodiments of the invention are described in the dependent claims.

### DESCRIPTION OF THE INVENTION

An imaging device according to the invention comprises a first polarizer and a second polarizer between which a strip of medication sachets can be arranged, a camera for imaging a first side of the strip of medication sachets through the second polarizer and means for changing the polarization direction of the first polarizer or the second polarizer.

The imaging device according to the invention can be used in a medication dispenser for imaging a strip of medication sachets. The strip of medication sachets comprises medication sachets attached one to another, where each medication sachet contains the medications that a patient should take at a prescribed date and time. Between the medication sachets there are seam areas along which the strip should be cut to separate the medication sachets from the strip. A seam area typically contains a heat seal. The separated medication sachets can be dispensed to the patient one medication sachet at a time.

The first polarizer and the second polarizer are linear polarizers. A linear polarizer is an optical filter that allows light waves of a specific polarization to pass through while blocking light waves of other polarizations. In other words, the linear polarizer works by transmitting light that oscillates in a particular direction (the polarization direction) and absorbing or reflecting light that oscillates in other directions. The purpose of the first polarizer and the second polarizer is to enhance the visibility of a seam area (heat seal). The polarization of the light on the seam area differs from that on the other areas. The first polarizer and the second polarizer are arranged in parallel and at a distance from each other. The distance between the first polarizer and the second polarizer can be, for example, 10-150 mm. A strip of medication sachets to be imaged is meant to be arranged between the first polarizer and the second polarizer in such a manner that a first side of the strip faces a first side of the second polarizer, and a second side of the strip faces a first side of the first polarizer.

In the imaging device according to the invention, the polarization direction of the first polarizer or the second polarizer can be changed with the means for changing the polarization direction. The polarization direction of the polarizer can be electrically or mechanically controllable. The means for changing the polarization direction of the first polarizer or the second polarizer may comprise an actuator for mechanically controlling the polarization direction by rotating the polarizer, or a control unit that is configured to electrically control the polarization direction by changing the electrical voltage or current that is supplied to the polarizer.

The camera is arranged to image the first side of the strip of medication sachets through the second polarizer. The camera is configured to capture images of the strip using different polarization directions of the first or the second polarizer. The imaging device may comprise means for processing the captured images to detect a seam area between medication sachets. The processing means may comprise a processor and a memory including computer program code, the memory and the computer program code being configured to, with the processor, process the captured images to detect a seam area between medication sachets. Other information, such as text printed on a medication sachet may also be recognized from the captured images. The camera is preferably a digital camera. One or more lenses and/or mirrors can be arranged in an optical path between the camera and the second polarizer.

An advantage of the imaging device according to the invention is that it enables to accurately detect a seam area between medication sachets.

According to an embodiment of the invention the means for changing the polarization direction of the first polarizer or the second polarizer is configured to change the polarization direction between a first polarization direction, where the polarization directions of the first polarizer and the second polarizer are perpendicular, and a second polarization direction, where the polarization directions of the first polarizer and the second polarizer are parallel.

When the polarization directions of the first polarizer and the second polarizer are perpendicular, the polarizers can block the passage of light from a second side of the first polarizer to the second side of the second polarizer, unless the polarization state of the light has changed due to interaction with the strip of medication sachets.

The areas where the polarization has changed appear lighter in the image. When the polarization directions of the first polarizer and the second polarizer are parallel, the polarizers allow light that oscillates in the second polarization direction to pass through the polarizers from the second side of the first polarizer to the second side of the second polarizer. If the polarization state of the light has changed due to interaction with the strip of medication sachets, these areas appear darker in the image.

The camera is preferably configured to capture images of the strip of medication sachets both in the case where the polarization directions of the first polarizer and the second polarizer are perpendicular and where the polarization directions of the first polarizer and the second polarizer are parallel. An advantage of this is that it improves the detection of a seam area between medication sachets.

According to an embodiment of the invention the imaging device comprises a first light source for illuminating a second side of the strip of medication sachets through the first polarizer. The first light source is arranged at the second side of the first polarizer. The amount and polarization state of the light received into the camera from the first light source depends on the polarization directions of the first polarizer and the second polarizer as well as the properties of the strip of medication sachets through which the light passes.

The first light source is preferably arranged to provide an essentially even light distribution on the second side of the strip. The first light source can be a light panel that is arranged in contact with or at a distance from the second side of the first polarizer. The first light source may comprise one or more LEDs. The LEDs may be arranged together in an LED array or matrix. The imaging device may comprise a control unit that is configured to switch the first light source on and off.

According to an embodiment of the invention the imaging device comprises a second light source for illuminating the first side of the strip of medication sachets. The second light source is preferably arranged in a space between the first polarizer and the second polarizer. Part of the light emitted by the second light source is reflected from the first side of the strip of medication sachets and passed through the second polarizer into the camera. The amount and polarization state of the light received into the camera from the second light source depends on the polarization direction of the second polarizer as well as the properties of the strip of medication sachets.

The second light source is preferably arranged to provide an essentially even light distribution on the first side of the strip. The second light source may comprise one or more LEDs. The LEDs may be arranged together in an LED array or matrix. The imaging device may comprise a control unit that is configured to switch the second light source on and off.

According to an embodiment of the invention the imaging device comprises a reflector for reflecting light from the second light source towards the first side of the strip of medication sachets, the reflector having an aperture through which the first side of the strip of medication sachets can be imaged. The reflector is preferably arranged in a space between the first polarizer and the second polarizer. The reflector is arranged in such a manner that its opening faces the first side of the first polarizer. The reflector is preferably shaped so that together with the second light source they provide an essentially even light distribution on the first side of the strip of medication sachets. The aperture through which the strip of medication sachets is imaged faces the first side of the second polarizer. The aperture can be, for example, circular or rectangular in shape. The diameter of a circular aperture can be, for example, 5-30 mm, and the length of each side of a rectangular aperture can be, for example, 5-30 mm.

According to an embodiment of the invention the reflector is a tunnel-shaped reflector. The part of the strip of medication sachets to be imaged can be arranged inside the tunnel-shaped reflector.

According to an embodiment of the invention the second light source is attached to an opening of the reflector. The second light source is preferably attached to the opening of the reflector in such a manner that a main part of the light emitted by the second light source is directed towards a reflecting surface of the reflector from which reflecting surface the light is reflected towards the first side of the strip of medication sachets.

According to an embodiment of the invention the imaging device comprises a transparent panel for supporting the strip of medication sachets. The transparent panel is arranged in a space between the first polarizer and the second polarizer to support the second side of the strip of medication sachets. The transparent panel can be made of, for example, glass or acrylic.

The present invention also relates to a medication dispenser for dispensing medications to a patient. The medication dispenser comprises at least one imaging device according to the invention.

The medication dispenser may comprise a chamber into which a strip of medication sachets can be inserted. The strip of medication sachets can be conveyed from the chamber to an outlet of the medication dispenser by using one or more conveying devices, such as a pair of rollers which are rotated in opposite directions by an electric motor. The medication dispenser can separate the medication sachets from the strip and dispense them to the patient one medication sachet at a time. For this purpose, the medication dispenser may comprise a cutting device that is configured to cut the strip in a transverse direction at seam areas that have been detected with the imaging device. The separated medication sachets are conveyed to the outlet from which the patient can take the medication sachets.

The present invention also relates to a method for detecting a seam area between medication sachets by using an imaging device that comprises a camera, a first polarizer and a second polarizer, wherein the second polarizer is arranged between the camera and the first polarizer. The method according to the invention comprises arranging a strip of medication sachets between the first polarizer and the second polarizer, capturing, with the camera, a first image of a first side of the strip of medication sachets, changing the polarization direction of the first polarizer or the second polarizer, capturing, with the camera, a second image of the first side of the strip of medication sachets, combining the first image and the second image into a false-colour image, and processing the false-colour image to detect the seam area.

In the method according to the invention, the camera captures the first image and the second image of the first side of the strip of medication sachets through the second polarizer. The polarization direction of the first polarizer or the second polarizer is changed between capturing the first and second images. Due to the polarizing effects and birefringence, the seam area is highlighted in the first and second images in different ways. In the method according to the invention, the first and second images are combined into the false-colour image. This is done by combining the first image and the second image using two different colour channels. If the first and second images are not monochromatic, they are converted into monochromatic images before the first and second images are combined into the false-colour image. The false-colour image can be a false-colour RG (red-green) image, a false-colour RB (red-blue) image, or a false-colour GB (green-blue) image. The false-colour image is processed to detect the seam area.

The method according to the invention can be used in a medication dispenser for detecting seam areas between medications sachets. After a seam area between two medication sachets has been detected, the strip can be cut in a transverse direction at the seam area with a cutting device.

An advantage of the method according to the invention is that it enables to accurately detect a seam area between medication sachets.

According to an embodiment of the invention the polarization directions of the first polarizer and the second polarizer are perpendicular when one of the first and second images is captured, and the polarization directions of the first polarizer and the second polarizer are parallel when the other of the first and second images is captured.

According to an embodiment of the invention the method comprises illuminating a second side of the strip of medication sachets through the first polarizer when the first image and the second image are captured. The imaging device may comprise a first light source for illuminating the second side of the strip of medication sachets, and a control unit that is configured to switch the first light source on and off.

According to an embodiment of the invention the method comprises capturing, with the camera, a third image of the first side of the strip of medication sachets, illuminating the first side of the strip of medication sachets when the third image is captured, instead of combining the first image and the second image into a false-colour image, combining the first, the second and the third image into a false-colour image, and processing the false-colour image to detect the seam area.

The first, the second and the third image are combined into the false-colour image using three different colour channels. If the first, the second and the third image are not monochromatic, they are converted into monochromatic images before the first, the second and the third image are combined into the false-colour image. The false-colour image can be a false-colour RGB (red-green-blue) image. The false-colour image is processed to detect the seam area.

The imaging device may comprise a second light source for illuminating the first side of the strip of medication sachets, and a control unit that is configured to switch the second light source on and off. The second light source is preferably switched off when the first image and the second image are captured.

If the imaging device comprises a first light source for illuminating the second side of the strip of medication sachets through the first polarizer, it is preferably switched off when the third image is captured.

According to an embodiment of the invention the method comprises recognising text in the third image by using a neural network based optical character recognition. The text in the third image may include patient and medication related information that may contain a patient identifier (e.g. name and/or identification number), and dispense date and time of the medications. The text may have been printed using a specific layout on the medication sachet, either directly on a surface of the medication sachet or on a label that is attached on the medication sachet.

According to an embodiment of the invention the false-colour image is processed by using a neural network. The neural network can be trained with a large number of images where the seam area is manually labelled. The neural network creates a heatmap describing where the seam area is located. The positioning of the cut on the strip of medication sachets is done based on the heatmap.

According to an embodiment of the invention the method comprises, before the step of processing the false-colour image to detect the seam area, reducing the resolution of the false-colour image. The resolution of the image is reduced to decrease the computational burden of the neural network.

The exemplary embodiments of the invention presented in this text are not interpreted to pose limitations to the applicability of the appended claims. The verb "to comprise" is used in this text as an open limitation that does not exclude the existence of also unrecited features. The features recited in the dependent claims are mutually freely combinable unless otherwise explicitly stated.

The exemplary embodiments presented in this text and their advantages relate by applicable parts to the imaging device as well as the method according to the invention, even though this is not always separately mentioned.

### BRIEF DESCRIPTION OF THE DRAWINGS

- Fig. 1: illustrates an imaging device according to a first embodiment of the invention,
- fig. 2: illustrates an imaging device according to a second embodiment of the invention,
- fig. 3: illustrates a medication dispenser according to an embodiment of the invention, and
- fig. 4: illustrates a flow diagram of a method according to an embodiment of the invention.

### DETAILED DESCRIPTION OF THE DRAWINGS

The same reference signs are used of the same or like components in different embodiments.

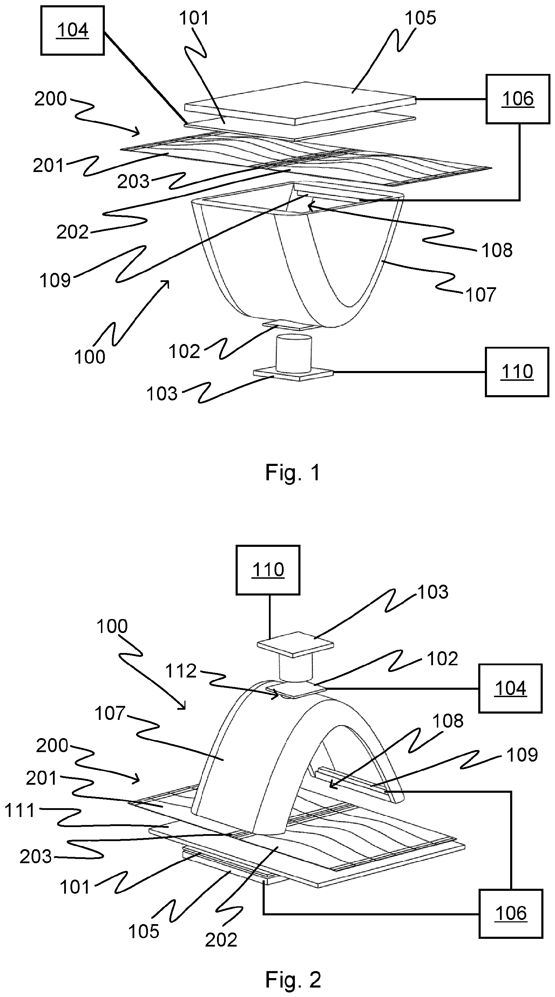

Fig. 1 illustrates an imaging device according to a first embodiment of the invention. The imaging device 100 is used for imaging a strip 200 of medication sachets. The strip 200 that is shown in fig. 1 consists of two medication sachets 201 and 202. Between the medication sachets 201 and 202 there is a seam area 203 along which the strip 200 should be cut to separate the medication sachets 201 and 202 from each other.

The imaging device 100 comprises two polarizers 101 and 102 between which the strip 200 is arranged, and a camera 103 for imaging a lower side of the strip 200 through the polarizer 102. The imaging device 100 comprises a control unit 104 that is connected to the polarizer 101 and configured to change the polarization direction of the polarizer 101 between a first polarization direction, where the polarization directions of the polarizers 101 and 102 are perpendicular, and a second polarization direction, where the polarization directions of the polarizers 101 and 102 are parallel.

The imaging device 100 comprises a light source 105 that is arranged at a distance from an upper side of the polarizer 101. The light source 105 is used for illuminating an upper side of the strip 200 through the polarizer 101. The amount and polarization state of the light received into the camera 103 from the light source 105 depends on the polarization directions of the polarizers 101 and 102 as well as the properties of the strip 200 through which the light passes. The imaging device 100 comprises a control unit 106 that is connected to the light source 105 and configured to switch the light source 105 on and off.

The imaging device 100 comprises a reflector 107 that is arranged in a space between the polarizers 101 and 102 in such a manner that its opening 108 faces the lower side of the strip 200, and a light source 109 that is attached to the opening 108 of the reflector 107. The reflector 107 is used for reflecting light from the light source 109 towards the lower side of the strip 200. Part of the light is reflected from the lower side of the strip 200 and passed through the polarizer 102 into the camera 103. The reflector 107 comprises an aperture (not shown in fig. 1) through which the lower side of the strip 200 is imaged. The amount and polarization state of the light received into the camera 103 from the light source 109 depends on the polarization direction of the polarizer 102 as well as the properties of the strip 200. The control unit 106 is connected to the light source 109 and configured to switch the light source 109 on and off.

The camera 103 is configured to capture images of the strip 200 using different polarization directions of the polarizer 101 and different settings of the light sources 105 and 109. The imaging device 100 comprises a processing unit 110 that is connected to the camera 103 and configured to process the captured images to detect the seam area 203, and to recognize text printed on the medication sachets 201 and 202.

Fig. 2 illustrates an imaging device according to a second embodiment of the invention. The imaging device 100 is used for imaging a strip 200 of medication sachets. The strip 200 that is shown in fig. 2 consists of two medication sachets 201 and 202. Between the medication sachets 201 and 202 there is a seam area 203 along which the strip 200 should be cut to separate the medication sachets 201 and 202 from each other. The strip 200 is placed on a transparent panel 111.

The imaging device 100 comprises two polarizers 101 and 102 between which the strip 200 is arranged, and a camera 103 for imaging an upper side of the strip 200 through the polarizer 102. The imaging device 100 comprises a control unit 104 that is connected to the polarizer 102 and configured to change the polarization direction of the polarizer 102 between a first polarization direction, where the polarization directions of the polarizers 101 and 102 are perpendicular, and a second polarization direction, where the polarization directions of the polarizers 101 and 102 are parallel.

The imaging device 100 comprises a light source 105 that is arranged in contact with a lower side of the polarizer 101. The light source 105 is used for illuminating a lower side of the strip 200 through the polarizer 101. The amount and polarization state of the light received into the camera 103 from the light source 105 depends on the polarization directions of the polarizers 101 and 102 as well as the properties of the strip 200 and the transparent panel 111 through which the light passes. The imaging device 100 comprises a control unit 106 that is connected to the light source 105 and configured to switch the light source 105 on and off.

The imaging device 100 comprises a reflector 107 that is arranged in a space between the polarizers 101 and 102 in such a manner that its opening 108 faces the upper side of the strip 200, and a light source 109 that is attached to the opening 108 of the reflector 107. The reflector 107 is used for reflecting light from the light source 109 towards the upper side of the strip 200. Part of the light is reflected from the upper side of the strip 200 and passed through the polarizer 102 into the camera 103. The reflector 107 comprises an aperture 112 through which the upper side of the strip 200 is imaged. The amount and polarization state of the light received into the camera 103 from the light source 109 depends on the polarization direction of the polarizer 102 as well as the properties of the strip 200. The control unit 106 is connected to the light source 109 and configured to switch the light source 109 on and off.

The camera 103 is configured to capture images of the strip 200 using different polarization directions of the polarizer 102 and different settings of the light sources 105 and 109. The imaging device 100 comprises a processing unit 110 that is connected to the camera 103 and configured to process the captured images to detect the seam area 203, and to recognize text printed on the medication sachets 201 and 202.

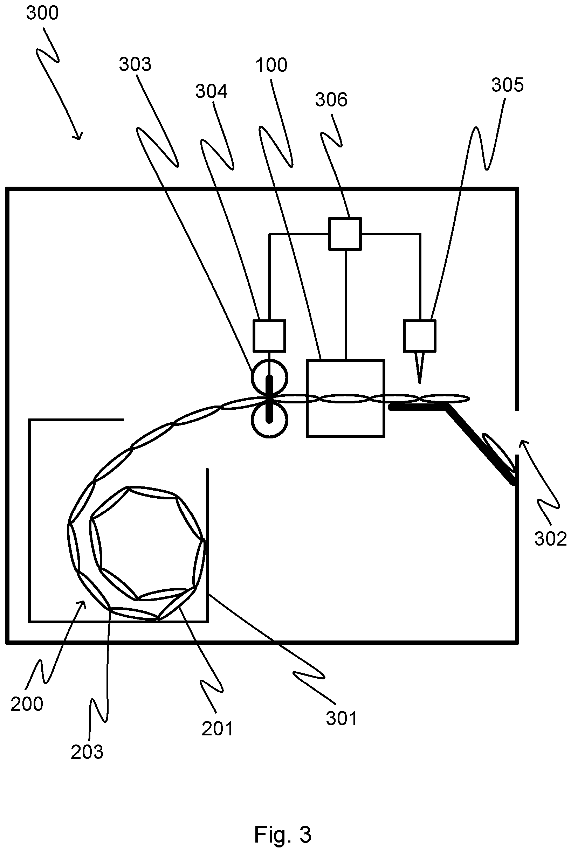

Fig. 3 illustrates a medication dispenser according to an embodiment of the invention for dispensing medications to a patient. The medication dispenser 300 comprises a chamber 301 into which a strip 200 of medication sachets has been inserted. The strip 200 consists of medication sachets 201 attached one to another. Each medication sachet 201 contains the medications that the patient should take at a prescribed date and time. The medication sachets 201 are conveyed from the chamber 301 to an outlet 302 of the medication dispenser 300 by using a pair of rollers 303 which are rotated in opposite directions by an electric motor 304.

The medication dispenser 300 comprises an imaging device 100 of fig. 2. In fig. 3, the imaging device 100 is shown schematically with a rectangle. The imaging device 100 is used for detecting seam areas 203 between the medication sachets 201, and for recognizing text printed on the medication sachets 201. The text may contain, for example, the dispensing date and time of the medications.

The medication dispenser 300 comprises a cutting device 305 that is configured to cut the strip 200 in a transverse direction at the seam areas 203 that have been detected with the imaging device 100. The separated medication sachets 201 are conveyed to the outlet 302 from which the patient can take the medication sachets 201.

The medication dispenser 300 comprises a control unit 306 that is connected to and configured to control the operation of the electric motor 304, the imaging device 100, and the cutting device 305.

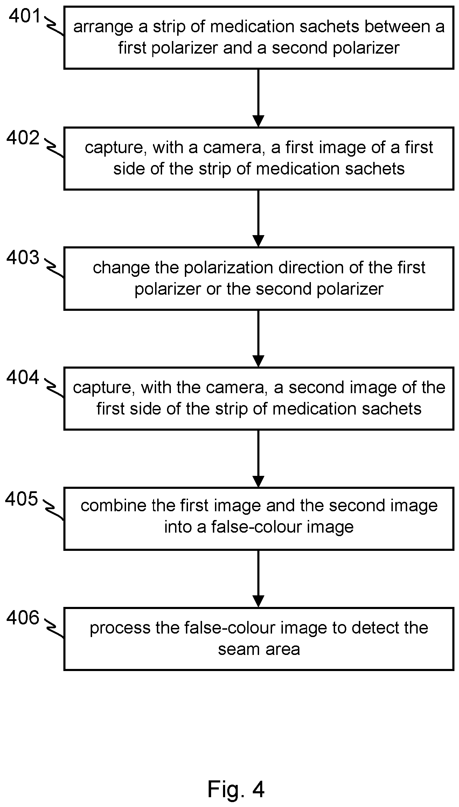

Fig. 4 illustrates a flow diagram of a method according to an embodiment of the invention for detecting a seam area between medication sachets. At step 401, a strip of medication sachets is arranged between a first polarizer and a second polarizer. At step 402, a first image of a first side of the strip of medication sachets is captured with a camera. The camera is arranged to image the strip of medication sachets through the second polarizer. At step 403, the polarization direction of the first polarizer or the second polarizer is changed. At step 404, a second image of the first side of the strip of medication sachets is captured with the camera. At step 405, the first image and the second image are combined into a false-colour image. At step 406, the false-colour image is processed to detect the seam area.

Only advantageous exemplary embodiments of the invention are described in the figures. It is clear to a person skilled in the art that the invention is not restricted only to the examples presented above, but the invention may vary within the limits of the claims presented hereafter. Some possible embodiments of the invention are described in the dependent claims, and they are not to be considered to restrict the scope of protection of the invention as such.

## Claims

1. An imaging device, **characterised in that** the imaging device comprises:
- a first polarizer and a second polarizer between which a strip of medication sachets can be arranged,
- a camera for imaging a first side of the strip of medication sachets through the second polarizer, and
- means for changing the polarization direction of the first polarizer or the second polarizer.

2. The imaging device according to claim 1, **characterised in that** the means for changing the polarization direction of the first polarizer or the second polarizer is configured to change the polarization direction between a first polarization direction, where the polarization directions of the first polarizer and the second polarizer are perpendicular, and a second polarization direction, where the polarization directions of the first polarizer and the second polarizer are parallel.

3. The imaging device according to claim 1 or 2, **characterised in that** the imaging device comprises a first light source for illuminating a second side of the strip of medication sachets through the first polarizer.

4. The imaging device according to any of the preceding claims, **characterised in that** the imaging device comprises a second light source for illuminating the first side of the strip of medication sachets.

5. The imaging device according to claim 4, **characterised in that** the imaging device comprises a reflector for reflecting light from the second light source towards the first side of the strip of medication sachets, the reflector having an aperture through which the first side of the strip of medication sachets can be imaged.

6. The imaging device according to claim 5, **characterised in that** the reflector is a tunnel-shaped reflector.

7. The imaging device according to claim 5 or 6, **characterised in that** the second light source is attached to an opening of the reflector.

8. The imaging device according to any of the preceding claims, **characterised in that** the imaging device comprises a transparent panel for supporting the strip of medication sachets.

9. A medication dispenser, **characterised in that** the medication dispenser comprises at least one imaging device according to any of the preceding claims.

10. A method for detecting a seam area between medication sachets by using an imaging device that comprises a camera, a first polarizer and a second polarizer, wherein the second polarizer is arranged between the camera and the first polarizer, **characterised in that** the method comprises:
- arranging a strip of medication sachets between the first polarizer and the second polarizer,
- capturing, with the camera, a first image of a first side of the strip of medication sachets,
- changing the polarization direction of the first polarizer or the second polarizer,
- capturing, with the camera, a second image of the first side of the strip of medication sachets,
- combining the first image and the second image into a false-colour image, and
- processing the false-colour image to detect the seam area.

11. The method according to claim 10, **characterised in that** the polarization directions of the first polarizer and the second polarizer are perpendicular when one of the first and second images is captured, and the polarization directions of the first polarizer and the second polarizer are parallel when the other of the first and second images is captured.

12. The method according to claim 10 or 11, **characterised in that** the method comprises illuminating a second side of the strip of medication sachets through the first polarizer when the first image and the second image are captured.

13. The method according to any of claims 10 to 12, **characterised in that** the method comprises:
- capturing, with the camera, a third image of the first side of the strip of medication sachets,
- illuminating the first side of the strip of medication sachets when the third image is captured,
- instead of combining the first image and the second image into a false-colour image, combining the first, the second and the third image into a false-colour image, and
- processing the false-colour image to detect the seam area.

14. The method according to claim 13, **characterised in that** the method comprises recognising text in the third image by using a neural network based optical character recognition.

15. The method according to any of claims 10 to 14, **characterised in that** the false-colour image is processed by using a neural network.

## Amended claims

### Amended claims in accordance with Rule 137(2) EPC.

1. An imaging device, **characterised in that** the imaging device comprises:
- a first polarizer and a second polarizer between which a strip of medication sachets can be arranged,
- a camera for imaging a first side of the strip of medication sachets through the second polarizer,
- means for changing the polarization direction of the first polarizer or the second polarizer, and
- a first light source for illuminating a second side of the strip of medication sachets through the first polarizer.

2. The imaging device according to claim 1, **characterised in that** the means for changing the polarization direction of the first polarizer or the second polarizer is configured to change the polarization direction between a first polarization direction, where the polarization directions of the first polarizer and the second polarizer are perpendicular, and a second polarization direction, where the polarization directions of the first polarizer and the second polarizer are parallel.

3. The imaging device according to claim 1 or 2, **characterised in that** the imaging device comprises a second light source for illuminating the first side of the strip of medication sachets.

4. The imaging device according to claim 3, **characterised in that** the imaging device comprises a reflector for reflecting light from the second light source towards the first side of the strip of medication sachets, the reflector having an aperture through which the first side of the strip of medication sachets can be imaged.

5. The imaging device according to claim 4, **characterised in that** the reflector is a tunnel-shaped reflector.

6. The imaging device according to claim 4 or 5, **characterised in that** the second light source is attached to an opening of the reflector.

7. The imaging device according to any of the preceding claims, **characterised in that** the imaging device comprises a transparent panel for supporting the strip of medication sachets.

8. A medication dispenser, **characterised in that** the medication dispenser comprises at least one imaging device according to any of the preceding claims.

9. A method for detecting a seam area between medication sachets by using an imaging device that comprises a camera, a first polarizer and a second polarizer, wherein the second polarizer is arranged between the camera and the first polarizer, **characterised in that** the method comprises:
- arranging a strip of medication sachets between the first polarizer and the second polarizer,
- capturing, with the camera, a first image of a first side of the strip of medication sachets,
- changing the polarization direction of the first polarizer or the second polarizer,
- capturing, with the camera, a second image of the first side of the strip of medication sachets,
- combining the first image and the second image into a false-colour image, and
- processing the false-colour image to detect the seam area.

10. The method according to claim 9, **characterised in that** the polarization directions of the first polarizer and the second polarizer are perpendicular when one of the first and second images is captured, and the polarization directions of the first polarizer and the second polarizer are parallel when the other of the first and second images is captured.

11. The method according to claim 9 or 10, **characterised in that** the method comprises illuminating a second side of the strip of medication sachets through the first polarizer when the first image and the second image are captured.

12. The method according to any of claims 9 to 11, **characterised in that** the method comprises:
- capturing, with the camera, a third image of the first side of the strip of medication sachets,
- illuminating the first side of the strip of medication sachets when the third image is captured,
- instead of combining the first image and the second image into a false-colour image, combining the first, the second and the third image into a false-colour image, and
- processing the false-colour image to detect the seam area.

13. The method according to claim 12, **characterised in that** the method comprises recognising text in the third image by using a neural network based optical character recognition.

14. The method according to any of claims 9 to 13, **characterised in that** the false-colour image is processed by using a neural network.
